# EUROPÄISCHE PATENTSCHRIFT

(11) **EP 2 138 098 B1**
(45) Veröffentlichungstag und Bekanntmachung des Hinweises auf die Patenterteilung: **29.06.2011**
(21) Anmeldenummer: 09157484.8
(22) Anmeldetag: 07.04.2009
(51) Int. Cl.: A61B 6/00, A61B 6/02, A61B 6/04

(54) **Mammographieanlage**
Mammography assembly
Installation de mammographie

(30) Priorität: 27.06.2008 DE 102008030698
(43) Veröffentlichungstag der Anmeldung: 30.12.2009
(73) Patentinhaber: Siemens AG, 80333 München (DE)
(72) Erfinder: Hanke, Wilhelm, 90607, Rückersdorf (DE); Mertelmeier,Dr. Thomas, 91058, Erlangen (DE)

(56) Entgegenhaltungen:
- EP-A- 1 419 735
- DE-A1-102006 048 607
- FR-A- 2 352 531

## Beschreibung

Die Erfindung betrifft eine Mammographieanlage mit einer Röntgenquelle, einem Detektor und einer im Strahlengang zwischen diesen angeordneten Kompressionsplatte sowie ein Verfahren zum Betrieb einer solchen Mammographieanlage.

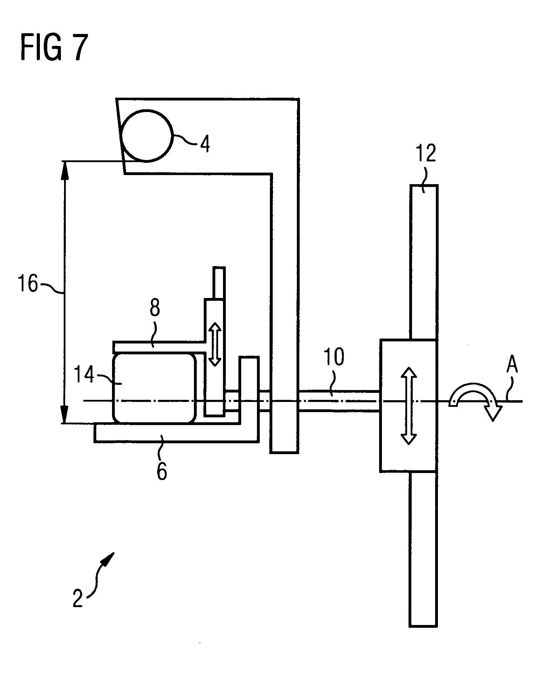

Eine herkömmliche Mammographieanlage 2 zeigt beispielhaft Fig. 7. Die Röntgenquelle 4, der Detektor 6 und die Kompressionsplatte 8 sind durch eine zentrale Welle 10 an einer Standsäule 12 gehalten. Bei der Röntgenquelle 4 handelt es sich um eine handelsübliche Röntgenröhre mit einer Wolfram-Drehanode. Der Detektor 6 umfasst eine nicht näher gezeigte Lagerplatte zur Ablage der zu untersuchenden Brust 14. Röntgenquelle 4, Detektor 6 und Kompressionsplatte 8 bilden gemeinsam das Messsystem der Mammographieanlage 2 und sind um eine gemeinsame Achse A gegenüber der Standsäule 12 drehbar. Zur Anpassung der Mammographieanlage 2 an die Größe des zu untersuchenden Patienten bzw. der zu untersuchenden Patientin wird das Messsystem entlang der Standsäule 12 verschoben. EP-A-1 419 735 beschreibt ein solches Gerät, wobei Röntgenquelle Kompressionsplatte und Detektor sich zusätzlich jeweils um eine eigene Schwenkachse drehen können. Im Folgenden wird stets auf eine Patientin Bezug genommen, gemeint sind jedoch stets weibliche und männliche Patienten.

Zur Untersuchung wird die Brust 14 zunächst komprimiert, dies erfolgt durch eine Verschiebung der Kompressionsplatte 8 in Strahlrichtung der Röntgenquelle 4, und anschließend von einem Bündel von Röntgenstrahlen durchleuchtet. Bei der in Fig. 7 gezeigten Mammographieanlage 2 ist der Abstand zwischen der Röntgenquelle 4 und dem Detektor 6, welcher im folgenden auch als Röhren-Detektor-Abstand bzw. abgekürzt RDA 16 (englisch: Source-Image-Distance, SID) bezeichnet wird, konstruktionsbedingt festgelegt. Als Röhren-Detektor-Abstand, RDA 16 wird die Distanz zwischen dem Ort der Röntgenstrahlerzeugung und dem Ort der Detektion der Röntgenstrahlen verstanden. Bei einem herkömmlichen Röntgengerät ist dies typischerweise der Abstand zwischen der Oberfläche der Anode der Röntgenröhre, von welcher das zur Untersuchung verwendete Röntgenstrahlbündel ausgeht, und dem röntgensensiblen Teil des Detektors, also beispielsweise eines Röntgenfilmes. Dieser Abstand wird auch als Fokus-Detektor-Abstand bezeichnet.

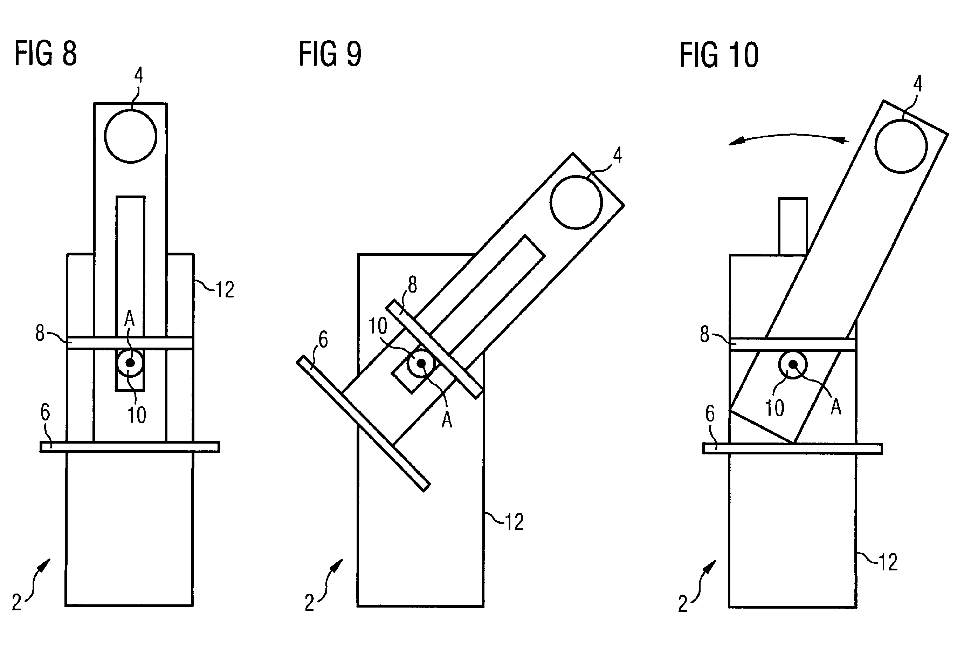

Mammographieaufnahmen können aus verschiedenen Richtungen, bei denen die Patientin jeweils eine andere Körperhaltung einnimmt, angefertigt werden. Solche Mammographieaufnahmen werden auch als Projektionen bezeichnet. Typisch sind die kranio-kaudale Projektion (CC-Projektion) oder die mediolateral-oblique Projektion (MLO-Projektion). Die Fig. 8 und 9 zeigen jeweils eine schematische Frontalansicht der Mammographieanlage 2. Fig. 8 zeigt beispielhaft die Aufnahmegeometrie für eine CC-Projektion, Fig. 9 zeigt beispielhaft die Aufnahmegeometrie für eine MLO-Projektion. Zum Wechsel der Projektionen wird das gesamte Messsystem um die zentrale Achse A geschwenkt.

Neben der herkömmlichen Mammographie gewinnt die Tomosynthese zunehmend an Bedeutung. Bei diesem Untersuchungsverfahren wird die in komprimiertem Zustand ortsfest gehaltene Brust 14 aus unterschiedlichen Richtungen (Projektionswinkeln) durchleuchtet. Zur Durchführung einer Tomosynthese ist es notwendig, dass die Kompression der Brust 14 von der Bewegung der Röntgenröhre 4 entkoppelt wird. Fig. 10 zeigt beispielhaft den Bewegungsablauf der Röntgenröhre 4 während der Aufnahme eines tomosynthetischen Bilddatensatzes. Während der Aufnahme stehen der Detektor 6 und die Kompressionsplatte 8 still, während sich die Röntgenröhre 4 bewegt.

Die mechanische Konstruktion, insbesondere einer für die Tomosynthese geeigneten Mammographieanlage 2, ist sehr aufwändig. Zum einen muss eine mechanisch stabile Aufnahme des Messsystems gewährleistet sein, wobei dieses zur Anpassung an die Größe der Patientin ebenfalls der Höhe nach verstellbar sein muss. Zur Einstellung der verschiedenen Projektionen (beispielsweise CC- oder MLO-Projektion) muss dass Messsystem zusätzlich drehbar an der Standsäule 12 befestigt sein. Soll die Mammographieanlage 2 außerdem für die Tomosynthese geeignet sein, so kommt eine zusätzliche Anforderung, nämlich die Entkopplung der Bewegung von Detektor 6 und Kompressionsplatte 8 von der Bewegung der Röntgenquelle 4 als mechanische Anforderung hinzu.

Aufgabe der vorliegenden Erfindung ist es, eine Mammographieanlage mit variabler Aufnahmegeometrie bei vereinfachter mechanischer Konstruktion sowie ein Betriebsverfahren für eine solche Mammographieanlage anzugeben.

Vorrichtungsbezogen wird die Aufgabe erfindungsgemäß gelöst durch eine Mammographieanlage mit den Merkmalen nach Anspruch 1.

Eine Mammographieanlage nach der Erfindung weist eine Röntgenquelle, einen Detektor und eine im Strahlengang zwischen diesen angeordnete Kompressionsplatte auf, und hat außerdem die folgenden Merkmale: Die Röntgenquelle, der Detektor und die Kompressionsplatte sind jeweils um eine eigene Schwenkachse schwenkbar an einer Standsäule gehalten. Die drei Schwenkachsen sind voneinander beabstandet, zumindest annähernd parallel zueinander und zumindest annähernd senkrecht zu einer Flächennormalen des Detektors orientiert. Die Röntgenquelle und die Kompressionsplatte sind in einer näherungsweise senkrecht zu ihrer Schwenkachse orientierten Ebene verschiebbar an der Standsäule gehalten.

Die Mammographieanlage mit den genannten Merkmalen ist gegenüber herkömmlichen Mammographieanlagen konstruktiv wesentlich vereinfacht. Anstatt einer gemeinsamen Welle, welche die Last des kompletten Messsystems (Röntgenquelle, Detektor, Kompressionsplatte) tragen muss, wird nun jeweils eine eigene Welle für jedes Bauteil des Messsystems verwendet. Aus diesem Grund ist es möglich, sowohl die tragende Welle selbst, als auch die dazugehörige Verstellmechanik auf eine geringere mechanische Belastung auszulegen. Bei herkömmlichen Mammographieanlagen wird bei einem Wechsel der Projektion (beispielsweise von der CC-Projektion in die MLO-Projektion) die gesamte Masse des Messsystems um eine einzige Achse gedreht. Bei einer solchen Drehbewegung fallen hohe Drehmomente an der Schwenkachse an. Bei einem manuellen Wechsel der Projektion belasten die hohen Drehmomente das Hilfspersonal, erfolgt der Wechsel automatisch, so müssen hohe Drehmomente von der Verstellmechanik aufgenommen werden. Bei einer Mammographieanlage mit den genannten Merkmalen sind die einzelnen Komponenten des Messsystems separat gelagert. Der Wechsel der Projektion erfolgt durch separate Bewegung der Bauteile des Messsystems. Die anfallenden Drehmomente sind aufgrund der vergleichsweise geringeren bewegten Masse ebenfalls wesentlich geringer. Das Bedienpersonal bzw. eine gegebenenfalls vorhandene motorische Verstellmechanik wird entlastet.

Vorteilhafte Ausgestaltungen der erfindungsgemäßen Mammographieanlage gehen aus den von Anspruch 1 abhängigen Unteransprüchen hervor.

Eine vielfach wünschenswerte Variation des Röhren-Detektor-Abstandes wird nach einer ersten Ausführungsform dadurch erreicht, dass die Röntgenquelle in eine Längsrichtung, die im Wesentlichen in Richtung der Längserstreckung der Standsäule weist, verschiebbar an dieser gehalten ist. Eine Variation des Röhren-Detektor-Abstandes erlaubt es die zur Bilderzeugung verwendete Dosis zu variieren.

Zum Wechsel der Projektion werden der Detektor, die Kompressionsplatte und die Röntgenquelle um ihre jeweilige Achse geschwenkt. Nach einem solchen Schwenkvorgang sind die einzelnen Komponenten des Messsystems - bezogen auf eine Oberflächennormale des Detektors - seitlich versetzt zueinander angeordnet. Dieser Versatz wird durch eine entsprechende Querverschiebung von Kompressionsplatte und Röntgenquelle kompensiert. Nach einer weiteren Ausführungsform ist daher die Röntgenquelle und/oder der Kompressionsplatte zusätzlich in eine von der Längsrichtung verschiedene Querrichtung verschiebbar an der Standsäule gehalten. Die Querrichtung ist dabei im Wesentlichen senkrecht zu der Schwenkachse der Röntgenquelle bzw. der Kompressionsplatte orientiert. Mit anderen Worten setzt sich die Verschiebebewegung der Röntgenquelle und/oder der Kompressionsplatte aus einer Verschiebung in Längsrichtung und einer Verschiebung in eine davon verschiedene Querrichtung zusammen.

Bei einer Veränderung der Aufnahmegeometrie, beispielsweise von einer CC-Projektion zu einer MLO-Projektion, verändert sich aus geometrischen Gründen der Röhren-Detektor-Abstand (RDA). Dieser nicht erwünschte Effekt wird dadurch kompensiert, dass die Röntgenquelle in Längsrichtung verfahren wird. Ein durch diese Korrektur in Längsrichtung wiederum entstehender Querversatz wird durch ein erneutes Verfahren in Querrichtung kompensiert.

Es sind im Wesentlichen zwei verschiedene Konstruktionen denkbar, um die oben dargelegte Verfahrmöglichkeiten für die Röntgenquelle und die Kompressionsplatte zu schaffen.

Eine erste Möglichkeit besteht nach einer Ausführungsform darin, dass die Röntgenquelle und/oder die Kompressionsplatte gemeinsam mit ihrer Schwenkachse in Längsrichtung verschiebbar an der Standsäule gehalten ist. Mit anderen Worten ist die Röntgenquelle und/oder die Kompressionsplatte derart an der Standsäule befestigt, dass deren Schwenkachse zwar der Verschiebung in Längsrichtung, nicht aber einer Verschiebung in Querrichtung folgt. Nach einer weiteren Möglichkeit und Ausführungsform ist die Röntgenquelle und/oder die Kompressionsplatte gemeinsam mit ihrer Schwenkachse in Längsrichtung und in Querrichtung verschiebbar an der Standsäule gehalten. Mit anderen Worten folgt die Schwenkachse sowohl einer Verschiebung in Längsrichtung als auch einer Verschiebung in Querrichtung.

Neben der klassischen Mammographie gewinnt die Tomosynthese als Untersuchungsmethode zunehmend an Bedeutung. Daher ist nach einer weiteren Ausführungsform die Röntgenquelle nach der Art eines Arrays von einer Vielzahl einzelner Röntgenemitter gebildet. Mit Hilfe einer solchen Multifokus-Röntgenquelle ist es möglich, die zu untersuchende Brust, ohne dass die Röntgenquelle selbst verfahren werden muss, aus einer Vielzahl verschiedener Projektionsrichtungen zu durchstrahlen. Zu diesem Zweck werden die einzelnen, annähernd nebeneinander angeordneten Röntgenemitter, nacheinander angesteuert und zur Emission angeregt. Durch den Einsatz einer solchen Multifokus-Röntgenröhre kann die Tomosyntheseuntersuchung wesentlich beschleunigt werden. Eine besonders geeignete Röntgenquelle, die eine Vielzahl einzelner Röntgenemitter umfasst, weist zumindest eine kalte Feldemissionskathode auf der Basis von Carbon-Nanotubes auf. Kathoden auf der Basis von Carbon-Nanotubes verwenden diese als Feldemitter, die Kathode muss also nicht geheizt werden, was insbesondere für eine Röntgenquelle, die eine Vielzahl einzelner Röntgenemitter umfasst vorteilhaft ist. Im Vergleich zu anderen Feldemittern, wie beispielsweise feinen Metallspitzen, sind Carbon-Nanotubes vergleichsweise robust.

Der Einsatz einer Multifokus-Röntgenröhre ist in Kombination mit einem variablen Röhren-Detektor-Abstand (RDA) besonders vorteilhaft, da durch eine Veränderung des RDA der Tomosynthesewinkel variiert werden kann. Wäre eine Veränderung des RDA nicht möglich, so ließe sich der Tomosynthesewinkel lediglich durch den Austausch der Röntgenquelle verändern. Diese müsste durch eine andere, verlängerte Röntgenquelle ausgetauscht werden. Ein solcher Austausch ist jedoch sehr aufwändig. Der Umbau der Mammographieanlage kann vorteilhaft durch die Variabilität des RDA vermieden werden.

Eine Variation des Röntgen-Detektor-Abstandes erlaubt es, die bildgebende Dosis zu variieren, ohne das die Röntgenleistung verändert werden muss. Dies ist besonders vorteilhaft, wenn die bildgebende Dosis erhöht werden soll, aber durch die Leistung der Röntgenquelle begrenzt ist. In diesem Fall kann der RDA verkleinert werden, d.h. der Tomosynthesewinkel vergrößert, und somit die bildgebende Dosis erhöht werden.

Eine Variation des RDA erlaubt eine zweifache Tomosyntheseabtastung. Die Brust wird zunächst bei einem ersten Röhren-Detektor-Abstand und anschließend bei einem zweiten von dem ersten verschiedenen Röhren-Detektor-Abstand abgetastet. Auf diese Weise kann die Abtastdichte erhöht werden, so dass für die Bildrekonstruktion Röntgenstrahlen berücksichtigt werden können, welche aus verschiedenen Richtungen die Brust durchstrahlen. In diesem Fall spricht man auch von einem Gewinn an zusätzlichen Fokusbahnen. Durch die in Folge der Variation des RDA gewonnenen zusätzlichen Informationen kann die Tiefenauslösung der Brust verbessert werden, bei der Tomosynthese unvermeidbar auftretende Artefakte werden reduziert.

Bei einer Veränderung der Aufnahmegeometrie, beispielsweise von einer CC-Projektion in eine MLO-Projektion, verändert sich aus geometrischen Gründen ebenfalls der RDA. Gemeinsam mit der Veränderung des RDA verändert sich auch der Tomosynthesewinkel. Um diesem gegebenenfalls unerwünschten Effekt zu kompensieren, ist es vorteilhaft, wenn die Röntgenquelle in eine Richtung, die näherungsweise der Längserstreckung der Standsäule entspricht, verstellt werden kann. Durch die Verstellung der Röntgenquelle kann der RDA (und somit auch der Tomosynthesewinkel) wieder auf den ursprünglichen Wert eingestellt werden.

Zur Anpassung der Mammographieanlage an die Größe der in der Regel stehend untersuchten Patientin, sind, nach einer weiteren Ausführungsform, die Röntgenquelle, der Detektor und die Kompressionsplatte gemeinsam in Längsrichtung verschiebbar. Die Mammographieanlage kann insbesondere so konstruiert sein, dass bei einer Anpassung an die Größe der Patientin, bei der im Wesentlichen die in den Detektor integrierte Lagerplatte ihrer Höhe nach verschoben wird, gleichzeitig die anderen Komponenten des Messsystems entsprechend verfahren werden. Die Handhabung der Mammographieanlage kann somit verbessert werden.

Hinsichtlich des Verfahrens wird die Aufgabe erfindungsgemäß gelöst durch ein Betriebsverfahren nach Anspruch 12.

Demnach wird zumindest ein erster tomosynthetischer Bilddatensatz bei einem ersten Röhren-Detektor-Abstand aufgenommen, ein zweiter tomosynthetischer Bilddatensatz wird bei einem zweiten, von dem ersten verschiedenen Röhren-Detektor-Abstand aufgenommen. Die Aufnahme zweier verschiedener tomosynthetischer Bilddatensätze aus jeweils unterschiedlichem Röhren-Detektor-Abstand, erlaubt es zusätzliche Bildinformationen zu gewinnen, und gegebenenfalls die aus den unterschiedlichen Röhren-Detektor-Abständen gewonnenen Bilddatensätze miteinander zu verrechnen. Durch die zusätzliche Abtastung der Brust mit einem weiteren Röhren-Detektor-Abstand werden bei der Tomosynthese auftretende Artefakte reduziert, die durch die inherent unvollständige Abtastung verursacht werden.

Weitere vorteilhafte Ausgestaltungen der erfindungsgemäßen Mammographieanlage sowie des erfindungsgemäßen Betriebsverfahrens gehen aus den vorstehenden nicht angesprochenen Unteransprüchen hervor. Die Erfindung wird im Folgenden unter Bezugnahme auf die Figuren der Zeichnungen weiter erläutert.

Es zeigen:
- Fig. 1: und 3 bis 6 Frontalansichten und
- Fig. 2: eine Seitenansicht einer Mammographieanlage gemäß zweier Ausführungsbeispiele,
- Fig. 7: eine Querschnittsansicht und
- Fig. 8 bis 10: Frontalansichten einer bekannten Mammographie- anlage.

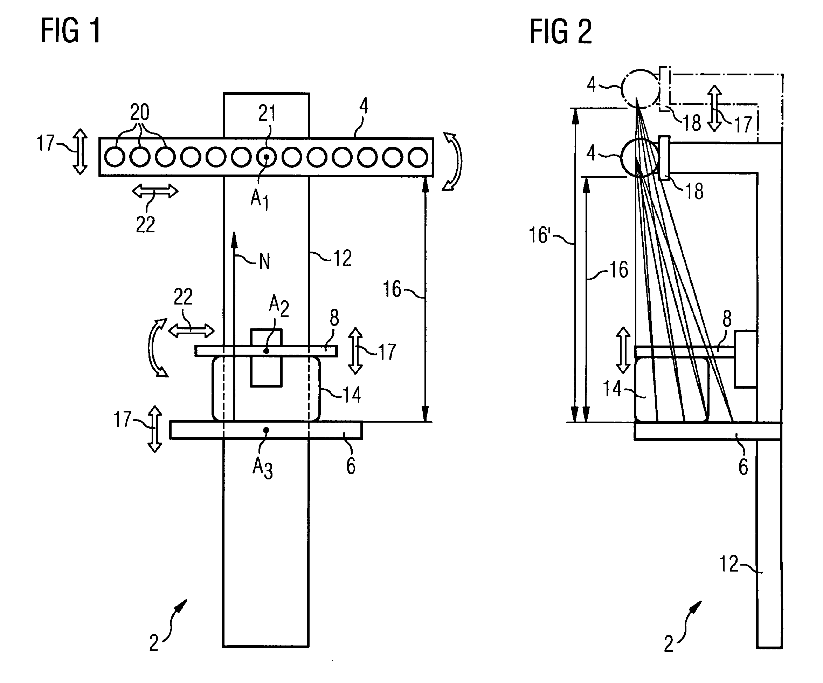

Fig. 1 zeigt eine Mammographieanlage 2 an deren Standsäule 12 eine Röntgenquelle 4, ein Detektor 6 und eine Kompressionsplatte 8 befestigt sind. Der Detektor 6 dient gleichzeitig als Lagerplatte zur Auflage der Brust 14. Die Röntgenquelle 4, der Detektor 6 und die Kompressionsplatte 8 bilden gemeinsam das Messsystem der Mammographieanlage 2, und sind, jedes Bauteil für sich, um eine Achse A₁-A₃ schwenkbar an der Standsäule 12 befestigt. Um eine Kompression der Brust 14 zu ermöglichen, ist die Kompressionsplatte 8 in eine Längsrichtung 17, die im wesentlichen in Längserstreckung der Standsäule 12 orientiert ist, an dieser verschiebbar gehalten. Gleiches trifft auf die Röntgenquelle 4 zu. Eine Verschiebung der Röntgenquelle 4 in eine Längsrichtung 17, die im Wesentlichen in Längserstreckung der Standsäule 12 orientiert ist, erlaubt Mammographieaufnahmen mit unterschiedlichem Röhren-Detektor-Abstand (RDA) 16.

Fig. 2 zeigt eine schematische Seitenansicht der Mammographieanlage 2 bei zwei verschiedenen Röhren-Detektor-Abständen 16, 16'. Die Röntgenquelle 4 ist auf einer Schiene 18 montiert, die eine Verschiebung der Röntgenquelle 4 in eine in Fig. 1 gezeigte Querrichtung 22 erlaubt. Auch die Kompressionsplatte 8 und der Detektor 6 können in eine Querrichtung 22 verschiebbar an der Standsäule 12 gehalten sein. Eine Verschiebung dieser Bauteile in Querrichtung 22 kann ebenso wie dies für die Röntgenquelle 4 dargestellt ist, mit Hilfe einer geeigneten Schiene erfolgen. Auf die Möglichkeit einer solchen Verschiebung wird weiter unten näher eingegangen.

Je nachdem, ob die Mammographieanlage 2 zur Aufnahme herkömmlicher Mammographien oder zur Aufnahme tomosynthetischer Bilddatensätze genutzt werden soll, handelt es sich bei der Röntgenquelle 4 entweder um eine herkömmliche Röntgenröhre oder um eine Multifokusröhre, welche aus einer Vielzahl einzelner Röntgenemitter 20 besteht.

In den Fig. 1 bis 6 ist eine Mammographieanlage 2 zur Aufnahme tomosynthetischer Bilddatensätze gezeigt. Die gezeigte Röntgenquelle 4 weist eine Vielzahl einzelner Röntgenemitter 20 auf, die nach der Art eines Arrays nebeneinander angeordnet sind. Vorzugsweise weisen die Röntgenemitter 20 eine kalte Feldemissionskathode auf der Basis von Carbon-Nanotubes auf. Für den Fall, dass die Mammographieanlage 2 für die Aufnahme herkömmlicher Mammographien genutzt werden soll, wird eine konventionelle Röntgenröhre unter Weglassen aller übrigen Röntgenemitter 20 an der in Fig. 1 nahe der Schwenkachse A₁ befindliche Position des Röntgenemitters 21 montiert.

Fig. 1 zeigt die Mammographieanlage 2 in einer Position, die die Aufnahme einer CC-Projektion erlaubt. Zum Wechsel der Projektion erfolgt eine Schwenkbewegung aller drei Komponenten des Messsystems um ihre jeweilige Schwenkachse A₁ bis A₃. Da nach einer solchen Schwenkbewegung, bezogen die Normale N des Detektors 6, die einzelnen Komponenten des Messsystems seitlich gegeneinander versetzt sind, werden die Röntgenquelle 4 und die Kompressionsplatte 8 jeweils in eine Querrichtung 22 verschoben. Diese Querrichtung 22 verläuft näherungsweise in einer Ebene senkrecht zu der jeweiligen Schwenkachse A₁,A₂ der Röntgenquelle 4 bzw. der Kompressionsplatte 8. Falls notwendig kann ebenfalls eine Verschiebung des Detektors 6, entsprechend der Verschiebung der Kompressionsplatte 8 und der Röntgenquelle 4, in Querrichtung 22 erfolgen, so dass der durch die Schwenkbewegung der einzelnen Komponenten, bezüglich der Normalen N hervorgerufene Versatz kompensiert werden kann.

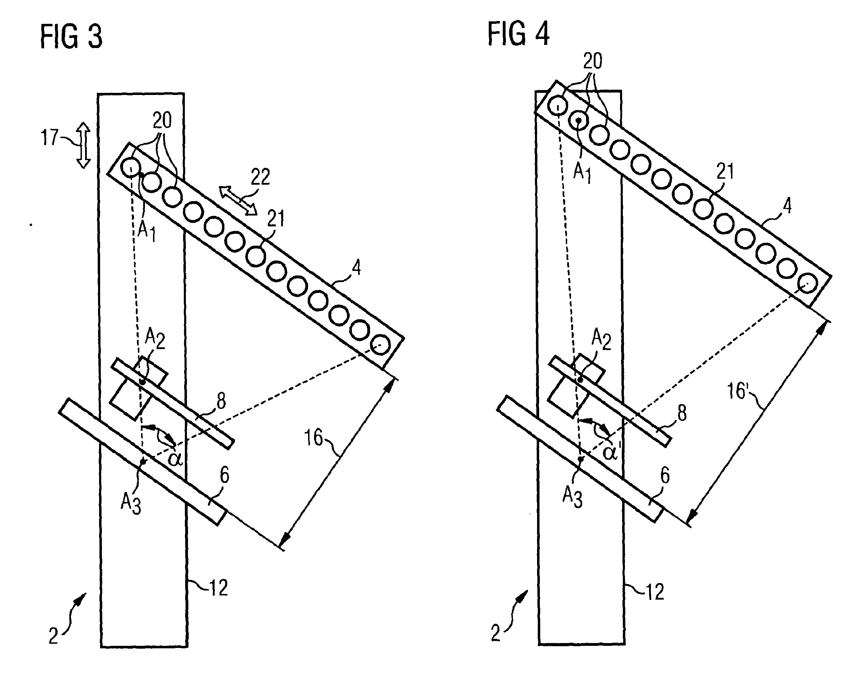

Das Ergebnis des Schwenkvorgangs und der anschließenden Querverschiebung ist in Fig. 3 gezeigt. Die Mammographieanlage 2 befindet sich nach erfolgtem Schwenkvorgang des Messsystems in einer Position für die Durchführung einer Mammographie in MLO-Projektion. Prinzipiell kann das Messsystem, solange seine Komponenten nicht miteinander kollidieren, auf beliebige Projektionen eingestellt werden. Vorzugsweise wird zur Veränderung des Aufnahmewinkels der Detektor 6 lediglich um seine Schwenkachse A₃ gedreht, die Kompressionsplatte 8 und die Röntgenquelle 4 hingegen werden sowohl um ihre Achsen A₂,A₃ gedreht, als auch in Querrichtung 22 verfahren.

In Folge des Schwenkvorgangs des Messsystems verändert sich der Abstand zwischen der Röntgenquelle 4 und dem Detektor 6, der Röhren-Detektor-Abstand, RDA 16. Durch die Veränderung des RDA 16 verändert sich auch der Tomosynthesewinkel α. Dieser mitunter unerwünschte Effekt wird ausgeglichen, indem die Röntgenquelle 4 in Längsrichtung 17, entlang der Standsäule 12 verfahren wird.

Fig. 4 zeigen die Mammographieanlage 2, bei nunmehr größerem RDA 16'. Entsprechend dem größeren RDA 16' ist der Tomosynthesewinkel α' kleiner als bei der in Fig. 3 gezeigten Position.

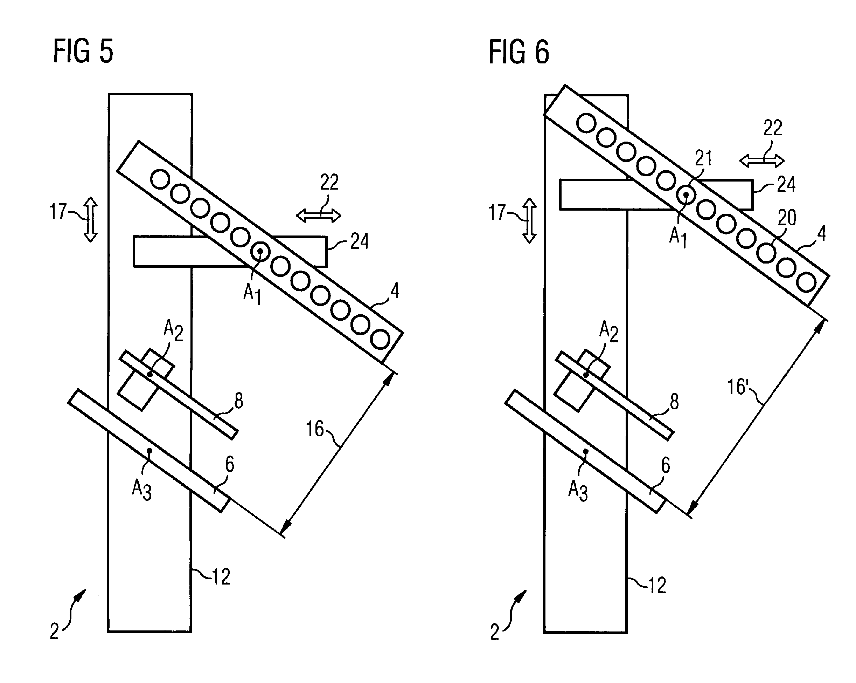

Fig. 5 und 6 zeigen ein weiteres Ausführungsbeispiel für eine Mammographieanlage 2. Diese befindet sich ähnlich wie die Mammographieanlage 2 in den Fig. 3 und 4 in einer Position zur Aufnahme einer MLO-Projektion. Im Gegensatz zu der in den Fig. 3 und 4 dargestellten Mammographieanlage 2 ist die Röntgenquelle 4 nunmehr an einem Ausleger 24 befestigt. Dieser erlaubt eine Bewegung der Röntgenquelle 4 in Querrichtung 22, die nun nahezu senkrecht zur Längserstreckung der Standsäule 12 orientiert ist. Die Röntgenquelle 4 ist so an dem Ausleger 24 befestigt, dass ihre Schwenkachse A₁ der Verschiebebewegung in Querrichtung 22 folgt. Im Gegensatz dazu folgt bei der in den Fig. 3 und 4 gezeigten Mammographieanlage 2 die Schwenkachse A₁ lediglich der Verschiebebewegung in Längsrichtung 17, nicht aber derjenigen in Querrichtung 22.

Auch bei der in den Fig. 5 und 6 gezeigten Mammographieanlage 2 ist es möglich, den RDA 16 zu verändern. Auch hier erfolgt die Veränderung des RDA 16, 16' durch eine Verschiebung der Röntgenquelle 4 in Längsrichtung 17.

Die Variation des RDA 16, 16' erlaubt ein besonderes Betriebsverfahren für eine Mammographieanlage 2 nach einem der besprochenen Ausführungsbeispiele. Bei diesem Betriebsverfahren wird ein erster tomosynthetischer Bilddatensatz bei einem ersten RDA 16 (vgl. Fig. 3 oder 5) und ein zweiter tomosynthetischer Bilddatensatz bei einem zweiten RDA 16' (vgl. Fig. 4 oder 6) erzeugt wird. Die beiden tomosynthetischen Bilddatensätze können einzeln ausgewertet werden oder anschließend zu einem gemeinsamen Bilddatensatz verrechnet werden.

### Bezugszeichenliste

- 2: Mammographieanlage
- 4: Röntgenquelle
- 6: Detektor
- 8: Kompressionsplatte
- 10: Welle
- 12: Standsäule
- 14: Brust
- 16,16': Röhren-Detektor-Abstand, RDA
- 17: Längsrichtung
- 18: Schiene
- 20, 21: Röntgenemitter
- 22: Querrichtung
- 24: Ausleger

- A: Schwenkachse
- N: Normale

- α,α': Tomosynthesewinkel

## Patentansprüche

1. Mammographieanlage (2) mit einer Röntgenquelle (4), einem Detektor (6) und einer im Strahlengang zwischen diesen angeordneten Kompressionsplatte (8), mit den folgenden Merkmalen:
- Röntgenquelle (4), Detektor (6) und Kompressionsplatte (8) sind jeweils um eine eigene Schwenkachse (A₁, A₂, A₃) schwenkbar an einer Standsäule (12) gehalten;
- die drei Schwenkachsen (A₁, A₂, A₃) sind voneinander beabstandet, zumindest annähernd parallel zueinander und zumindest annähernd senkrecht zu einer Flächennormalen (N) des Detektors (6) orientiert;
- die Kompressionsplatte (8) ist in einer senkrecht zu ihrer Schwenkachse (A₂) orientierten Ebene verschiebbar an der Standsäule (12) gehalten; **dadurch gekennzeichnet, dass** auch die Röntgenquelle (4) in einer senkrecht zu ihrer Schwenkachse (A₁) orientierten Ebene verschiebbar an der Standsäule (12) gehalten ist.

2. Mammographieanlage (2) nach Anspruch 1, bei der die Röntgenquelle (4) in eine Längsrichtung (17) verschiebbar an der Standsäule (12) gehalten ist, wobei die Längsrichtung (17) im Wesentlichen in Richtung der Längserstreckung der Standsäule (12) weist.

3. Mammographieanlage (2) nach Anspruch 2, bei der die Röntgenquelle (4) zusätzlich in eine von der Längsrichtung (17) verschiedene Querrichtung (22), die im Wesentlichen senkrecht zu der Schwenkachse (A₁) der Röntgenquelle (4) orientiert ist, verschiebbar an der Standsäule (12) gehalten ist.

4. Mammographieanlage (2) nach Anspruch 3, bei der die Röntgenquelle (4) gemeinsam mit ihrer Schwenkachse (A₁) in Längsrichtung (17) verschiebbar an der Standsäule (12) gehalten ist.

5. Mammographieanlage (2) nach Anspruch 3, bei der die Röntgenquelle (4) gemeinsam mit ihrer Schwenkachse (A₁) in Längsrichtung (17) und in Querrichtung (22) verschiebbar an der Standsäule (12) gehalten ist.

6. Mammographieanlage (2) nach einem der vorstehenden Ansprüche, bei der die Kompressionsplatte (6) in eine Längsrichtung (17), die im Wesentlichen in Richtung der Längserstreckung der Standsäule (12) weist, und zusätzlich in eine von der Längsrichtung (17) verschiedene Querrichtung (22), die im Wesentlichen senkrecht zu der Schwenkachse (A₂) der Kompressionsplatte (6) orientiert ist, verschiebbar an der Standsäule (12) gehalten ist.

7. Mammographieanlage (2) nach Anspruch 6, bei der die Kompressionsplatte (6) gemeinsam mit ihrer Schwenkachse (A₂) in Längsrichtung (17) verschiebbar an der Standsäule (12) gehalten ist.

8. Mammographieanlage (2) nach Anspruch 6, bei der die Kompressionsplatte (6) gemeinsam mit ihrer Schwenkachse (A₂) in Längsrichtung (17) und in Querrichtung (22) verschiebbar an der Standsäule (12) gehalten ist.

9. Mammographieanlage (2) nach einem der vorherstehenden Ansprüche, bei der die Röntgenquelle (4), der Detektor (6) und die Kompressionsplatte (8) derart an der Standsäule (12) befestigt sind, so dass diese gemeinsam in Längsrichtung (17) verschiebbar sind.

10. Mammographieanlage (2) nach einem der vorstehenden Ansprüche, bei der die Röntgenquelle (4) nach der Art eines Array von einer Vielzahl einzelner Röntgenemitter (20, 21) gebildet ist.

11. Mammographieanlage (2) nach Anspruch 10, bei der zumindest ein Röntgenemitter (20, 21) eine kalte Feldemissionskathode auf der Basis von Carbon-Nanotubes umfasst.

12. Verfahren zum Betrieb einer Mammographieanlage (2) nach einem der vorstehenden Ansprüche, bei dem zumindest ein erster tomosynthetischer Bilddatensatz bei einem ersten Röhren-Detektor-Abstand (16) und zumindest ein zweiter tomosynthetischer Bilddatensatz bei einem zweiten, von dem ersten verschiedenen Röhren-Detektor-Abstand (16') aufgenommen wird.

13. Verfahren zum Betrieb einer Mammographieanlage (2) nach Anspruch 12, bei dem der erste und zweite tomosynthetische Bilddatensatz zur Bildrekonstruktion herangezogen werden.

## Claims

1. Mammography system (2) having an X-ray source (4), a detector (6) and a compression plate (8) disposed in the beam path between these two, having the following features:
- X-ray source (4), detector (6) and compression plate (8) are held on a vertical column (12) in such manner that they can be pivoted respectively about their own pivot axis (A₁, A₂, A₃);
- the three pivot axes (A₁, A₂, A₃) are spaced apart from one another and oriented at least substantially parallel to one another and at least substantially perpendicular to a surface normal (N) of the detector (6);
- the compression plate (8) is held on the vertical column (12) in such a manner that it can be displaced in a plane oriented perpendicular to its pivot axis (A₂),
**characterised in that** the X-ray source (4) is also held on the vertical column (12) in such a manner that it can be displaced in a plane oriented perpendicular to its pivot axis (A₁).

2. Mammography system (2) according to claim 1, wherein the X-ray source (4) is held on the vertical column (12) in such a manner that it can be displaced in a longitudinal direction (17), the longitudinal direction (17) pointing essentially in the direction of the longitudinal extension of the vertical column (12).

3. Mammography system (2) according to claim 2, wherein the X-ray source (4) is held on the vertical column (12) in such a manner that it can also be displaced in a transverse direction (22), which is different from the longitudinal direction (17) and which is oriented essentially perpendicular to the pivot axis (A₁) of the X-ray source (4).

4. Mammography system (2) according to claim 3, wherein the X-ray source (4) is held on the vertical column (12) in such a manner that it can be displaced in a longitudinal direction (17) together with its pivot axis (A₁).

5. Mammography system (2) according to claim 3, wherein the X-ray source (4) is held on the vertical column (12) in such a manner that it can be displaced in a longitudinal direction (17) and in a transverse direction (22) together with its pivot axis (A₁).

6. Mammography system (2) according to one of the preceding claims, wherein the compression plate (6) is held on the vertical column (12) in such a manner that it can be displaced in a longitudinal direction (17), which essentially points in the direction of the longitudinal extension of the vertical column (12), and additionally in a transverse direction (22), which is different from the longitudinal direction (17) and is oriented essentially perpendicular to the pivot axis (A₂) of the compression plate (6).

7. Mammography system (2) according to claim 6, wherein the compression plate (6) is held on the vertical column (12) in such a manner that it can be displaced in a longitudinal direction (17) together with its pivot axis (A₂).

8. Mammography system (2) according to claim 6, wherein the compression plate (6) is held on the vertical column (12) in such a manner that it can be displaced in a longitudinal direction (17) and in a transverse direction (22) together with its pivot axis (A₂).

9. Mammography system (2) according to one of the preceding claims, wherein the X-ray source (4), the detector (6) and the compression plate (8) are fastened to the vertical column (12) in such a manner that they can be displaced together in a longitudinal direction (17).

10. Mammography system (2) according to one of the preceding claims, wherein the X-ray source (4) is formed in the manner of an array of a plurality of individual X-ray emitters (20, 21).

11. Mammography system (2) according to claim 10, wherein the at least one X-ray emitter (20, 21) comprises a carbon nanotube-based cold field emission cathode.

12. Method for operating a mammography system (2) according to one of the preceding claims, wherein at least a first tomosynthetic image data set is recorded at a first tube-detector distance (16) and at least a second tomosynthetic image data set is recorded at a second tube-detector distance (16'), which is different from the first.

13. Method for operating a mammography system (2) according to claim 12, wherein the first and second tomosynthetic image data sets are used for image reconstruction.

## Revendications

1. Installation de mammographie (2) comprenant une source de rayons X (4), un détecteur (6) et une plaque de compression (8) disposée dans la trajectoire du faisceau entre ces éléments, présentant les caractéristiques suivantes :
- la source de rayons X (4), le détecteur (6) et la plaque de compression (8) sont maintenus sur une colonne verticale (12) de façon à pouvoir basculer à chaque fois autour d'un axe de pivotement (A₁, A₂, A₃ propre ;
- les trois axes de pivotement (A₁, A₂, A₃) sont espacés les uns des autres, orientés au moins approximativement parallèlement entre eux et au moins approximativement perpendiculairement à une verticale de surface (N) du détecteur (6) ;
- la plaque de compression (8) est maintenue sur la colonne verticale (12) de façon à pouvoir coulisser dans un plan orienté perpendiculairement à son axe de pivotement (A₂) ; **caractérisé en ce qu'**également la source de rayons X (4) est maintenue sur la colonne verticale (12) de façon à pouvoir coulisser dans un plan orienté perpendiculairement à son axe de pivotement (A₁).

2. Installation de mammographie (2) selon la revendication 1, sur laquelle la source de rayons X (4) est maintenue sur la colonne verticale (12) de façon coulissante dans une direction longitudinale (17), la direction longitudinale (17) étant dirigée principalement en direction de l'extension longitudinale de la colonne verticale (12).

3. Installation de mammographie (2) selon la revendication 2, sur laquelle la source de rayons X (4) est maintenue additionnellement de façon coulissante sur la colonne verticale (12) dans une direction transversale (22) différente de la direction longitudinale (17), qui est orientée sensiblement perpendiculairement à l'axe de pivotement (A₁) de la source de rayons X (4).

4. Installation de mammographie (2) selon la revendication 3, sur laquelle la source de rayons X (4) est maintenue sur la colonne verticale (12) de façon coulissante dans la direction longitudinale (17) conjointement avec son axe de pivotement (A₁).

5. Installation de mammographie (2) selon la revendication 3, sur laquelle la source de rayons X (4) est maintenue sur la colonne verticale (12) de façon coulissante conjointement avec son axe de pivotement (A₁) dans la direction longitudinale (17) et dans la direction transversale (22).

6. Installation de mammographie (2) selon l'une quelconque des revendications précédentes, sur laquelle la plaque de compression (6) est maintenue sur la colonne verticale (12) de façon à pouvoir coulisser dans une direction longitudinale (17), qui est dirigée sensiblement en direction de l'extension longitudinale de la colonne verticale (12), et en supplément dans une direction transversale (22) différente de la direction longitudinale (17), qui est orientée sensiblement perpendiculairement à l'axe de pivotement (A₂) de la plaque de compression (6).

7. Installation de mammographie (2) selon la revendication 6, sur laquelle la plaque de compression (6) est maintenue sur la colonne verticale (12) de façon à pouvoir coulisser conjointement avec son axe de pivotement (A₂) dans la direction longitudinale (17).

8. Installation de mammographie (2) selon la revendication 6, sur laquelle la plaque de compression (6) est maintenue sur la colonne verticale (12) de façon à pouvoir coulisser conjointement avec son axe de pivotement (A₂) dans la direction longitudinale (17) et dans la direction transversale (22).

9. Installation de mammographie (2) selon l'une quelconque des revendications précédentes, sur laquelle la source de rayons X (4), le détecteur (6) et la plaque de compression (8) sont fixées sur la colonne verticale (12) de telle sorte que ces éléments peuvent coulisser conjointement dans la direction longitudinale (17).

10. Installation de mammographie (2) selon l'une quelconque des revendications précédentes, sur laquelle la source de rayons X (4) est formée à la façon d'un appareil par une pluralité d'émetteurs de rayons X (20, 21) individuels.

11. Installation de mammographie (2) selon la revendication 10, sur laquelle au moins un émetteur de rayons X (20, 21) comprend une cathode d'émission par champ électrique froid sur la base de nanotubes de carbone.

12. Procédé pour faire fonctionner une installation de mammographie (2) selon l'une quelconque des revendications précédentes, dans lequel au moins un premier ensemble de données d'image tomosynthétique est enregistré à une première distance tube-détecteur (16) et au moins un second ensemble de données d'image tomosynthétique est enregistré à une seconde distance tube-détecteur (16') différente de la première distance.

13. Procédé pour faire fonctionner une installation de mammographie (2) selon la revendication 12, dans lequel le premier et le second ensembles de données d'image tomosynthétique sont utilisés pour la reconstitution de l'image.
